# EUROPÄISCHE PATENTSCHRIFT

(11) **EP 0 890 327 B1**
(45) Veröffentlichungstag und Bekanntmachung des Hinweises auf die Patenterteilung: **27.08.2003**
(21) Anmeldenummer: 98107100.4
(22) Anmeldetag: 18.04.1998
(51) Int. Cl.: A45D 34/02

(54) **Behälter in einem im Behälter befindichen Produkt**
Container with a product inside
Récipient avec un produit dedans

(30) Priorität: 20.06.1997 DE 19726179
(43) Veröffentlichungstag der Anmeldung: 13.01.1999
(73) Patentinhaber: Wella Aktiengesellschaft, 64274 Darmstadt (DE)
(72) Erfinder: Steigerwald, Franz, 64347 Griesheim (DE); Eberhardt, Heiko, 61440 Oberursel (DE); Stähle, Liane, 64372 Ober-Ramstadt (DE); Burghaus, Johannes, 64823 Gross-Umstadt (DE)

(56) Entgegenhaltungen:
- WO-A-87/03563
- CA-A- 983 437
- US-A- 2 241 022
- US-A- 3 951 622
- PATENT ABSTRACTS OF JAPAN vol. 014, no. 310 (C-0736), 4. Juli 1990 -& JP 02 107266 A (NISSO KAGAKU KK), 19. April 1990

## Beschreibung

Die Erfindung betrifft einen Behälter mit einem im Behälter befindlichen Produkt, wobei die Wand des Behälters mindestens eine abgeschlossene Kammer begrenzt und sich in der Kammer eine Substanz mit einem Duftstoff befindet, der durch eine duftstoffdurchlässige Kammerwand in die Umgebung des Behälters diffundiert, die Kammer in die Wand integriert ist, das Produkt den gleichen Duftstoff aufweist wie die Substanz, und die Kammerwand eine schnellere Permeation als die Wand zuläßt.

Ein aus dem DE-G 8490017.2 bekannter Behälter enthält eine aktive Duftsubstanz in einer Kammer mit einer aus einem polymeren Material hergestellten Wand. Die Wand erlaubt ein Diffundieren der Dämpfe der Duftsubstanz in die umgebende Luft, um diese zu parfümieren.

Der bekannte Behälter hat den Nachteil, daß er zur Aufbewahrung eines kosmetischen, einen Duftstoff enthaltenden Produktes wenig geeignet ist, da ein erhöhter Verlust des Duftstoffes an die Umgebung bei diesem Behälter vorkommt, und dadurch die Qualität des kosmetischen Produktes verringert wird.

Aus der US 2,241,022 ist eine Flasche zum Aufbewahren einer flüchtigen Flüssigkeit, insbesondere eines Parfums, bekannt. Im Verschluß der Flasche befindet sich in einer Kammer ein saugfähiges Material. Dieses nimmt Substanzen auf, welche dem Inhalt der Flasche ähnlich sind, um einem Kunden eine Duftprobe bzgl. des Inhalts der Flasche anzubieten. Nach außen hin ist das Material von einer Kammerwand begrenzt, welche mehrere Löcher aufweist. Durch diese Löcher gelangt der Duft an die äußere Umgebung.

Auch diese Flasche hat den Nachteil, daß ein erhöhter Verlust von Duftstoff vorkommt. Dieser hohe Verlust führt dazu, daß die Duftintensität relativ schnell abnimmt.

Der Erfindung liegt die Aufgabe zu Grunde, einen Behälter der eingangs genannten Art mit einem im Behälter befindlichen Produkt derart auszugestalten, daß er zur Aufbewahrung von kosmetischem Produkt geeignet ist, und ein Verbraucher über einen relativ langen Zeitraum am Behälter eine Information über einen im Produkt befindlichen Duftstoff erhält.

Gelöst ist die Aufgabe gemäß dem kennzeichnenden Teil des Anspruchs 1. Danach ist die Kammerwand ein Etikett.

Die Erfindung hat den Vorteil, daß der Behälter zur Aufbewahrung von kosmetischem Produkt geeignet ist, und daß die Wand des Behälters aus herkömmlichem, für kosmetische Produkte geeignetem Material möglich ist. Das nach außen, vom Behälter weg die Kammer begrenzende Etikett ist, verglichen mit der Wand, in erhöhtem Maße duftstoffdurchlässig. Damit gelangt quasi nur der von der in der Kammer befindlichen Substanz abgegebene Duftstoff nach außen. Der Duftstoff des Produktes wird kaum durch die Wand abgegeben.
Ein Verbraucher erhält eine Information über den Duftstoff des im Behälter befindlichen Produktes, da das Produkt den gleichen Duftstoff aufweist wie die Substanz, und der Verbraucher den Duftstoff der Substanz sensorisch wahrnehmen kann. Diese Wahrnehmung ist über einen relativ langen Zeitraum möglich.

Da die Kammer vollständig in die Wand integriert ist, wird die äußere Erscheinung des Behälters durch die Kammer nicht beeinträchtigt.

Weitere vorteilhafte Ausgestaltungen der Erfindung sind in den Ansprüchen 2 bis 6 beschrieben.

Die Kammerwand kann aus Polypropylen oder Polyethylen niedriger Dichte bestehen (Anspruch 2). Ist als Material für das Etikett das gleiche Material wie für die Wand vorgesehen, und ist das Etikett deutlich dünner als die zum Inneren des Behälters hin gelegene, die Kammer begrenzende Wandung (Anspruch 6), so ist die Duftstoffdiffusion durch die Kammerwand deutlich höher als durch die Wandung. Eine Diffusion durch die Wandung ist insofern kein Nachteil, als der Duftstoff aus der Kammer derart zum Produkt gelangt. Eine vergleichsweise zur Kammerwand relativ dicke Wand bzw. Wandung dient aber einer Verhinderung ungewünschter Diffusion des im Behälter befindlichen Duftstoffs nach außen.

Als Kammerwand eignet sich auch ein gesintertes Blättchen (Anspruch 3), durch dessen Poren der Duftstoff nach außen gelangt.

Weise in eine Kammer eingefüllt werden kann, und das eine starke Duftstoffabgabe hat.

Ist die Kammerwand Teil einer Verschlußkappe (Anspruch 5), so befindet sich die Kammer an einer exponierten Stelle des Behälters und ist somit für einen Verbraucher in vorteilhafter Weise sensorisch erkennbar.

Im folgenden wird die Erfindung anhand Ausführungsbeispiele darstellender Figuren näher beschrieben. Es zeigt:
- Figur 1: in einem Vertikalschnitt eine Wand eines Behälters mit einer Kammer, in die als Substanz und Duftstoff ein Parfümöl in konzentrierter Form eingelegt ist, und die mittels eines Etiketts verschlossen ist;
- Figur 2: in einer Seitenansicht mit teilweisem Vertikalschnitt einen Behälter mit einer Vielzahl an Kammern, wobei nur im Vertikalschnitt und in einem kreisförmigen Sektor die Kammern zeichnerisch dargestellt wurden;
- Figur 3: in einer Seitenansicht einen Behälter mit einem Etikett und einer einzigen Kammer, wobei die Kammer vom Etikett verschlossen ist, sowie
- Figur 4: in einer Seitenansicht mit teilweisem Vertikalschnitt einen Behälter, in dessen Verschlußkappe eine mittels eines Etiketts verschlossene Kammer ausgespart ist.

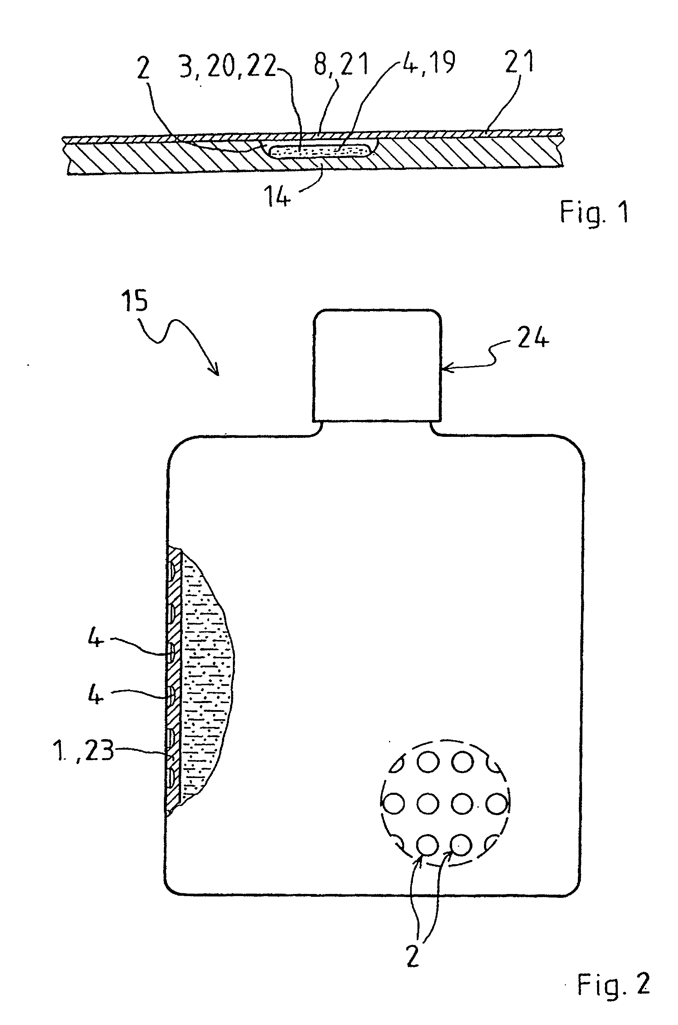

In einem Behälter befindet sich ein kosmetisches Produkt, in welchem ein Duftstoff vorkommt. Der Duftstoff des Produktes ist der gleiche Duftstoff wie der Duftstoff 4 in einer flüssigen Substanz 3 einer Kammer 2 des Behälters (Figur 1). Die verschlossene Kammer 2 dient als Riechprobe für einen Kunden. Der Kunde kann den durch die dünne Kammerwand 8 diffundierenden Duftstoff 4 sensorisch erfassen. Er erfährt somit den im Behälter enthaltenen Duftstoff, ohne den Behälter öffnen zu müssen. Der Duftstoff im Produkt diffundiert quasi nicht durch die relativ dicke Wandung 14 und die noch dickere Wand, wodurch das Produkt den Duftstoff 4 quasi nicht verliert und somit qualitätsstabil im Behälter aufbewahrt werden kann.

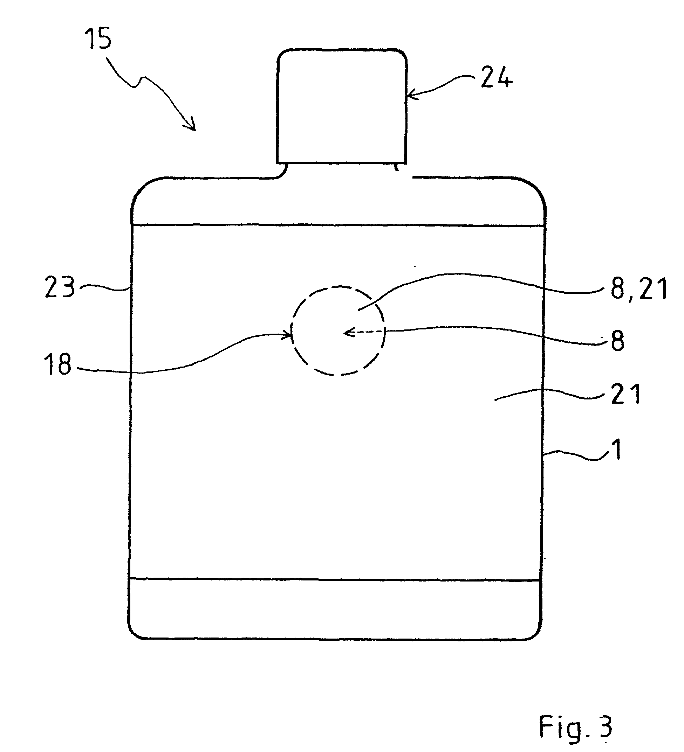

Die Kammer 2 enthält als Substanz 3 und Duftstoff 4 ein Parfümöl 19 in konzentrierter Form 20. Die Kammerwand 8 wird durch ein Etikett 21 gebildet, welches auf die Wand des Behälters geklebt ist. Die Substanz 3 ist eine Flüssigkeit 22. Ein Behälter 15 kann eine Vielzahl an Kammern 2 aufweisen (Figur 2) und dadurch eine gleichmäßige Abgabe von Duftstoff 4 an seinem Mantel 23 ermöglichen. Es kann aber auch nur eine einzige Riechprobe am Behälter 15 vorgesehen sein. Beim Ausführungsbeispiel der Figur 3 ist derart nur eine einzige, dafür aber relativ große Kammer 8 am Mantel 23 eines Behälters 15 vorgesehen. Die Kammer 8 wird von einem umlaufenden Etikett 21 als Kammerwand 8 verschlossen.

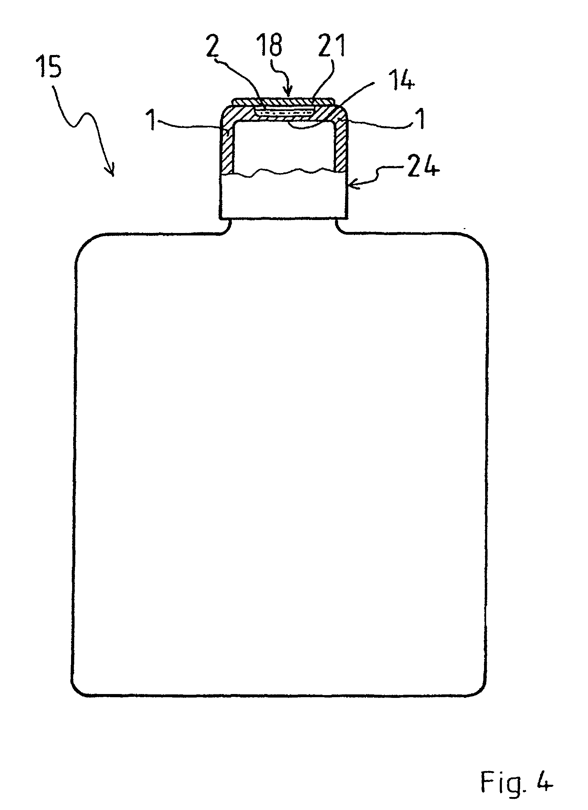

Beim Ausführungsbeispiel der Figur 4 ist schließlich die Kammer 2 in der Wand 1 einer Verschlußkappe 24 integriert und mittels eines aufgeklebten Etiketts 21 verschlossen. An den Behältern 15 der Figuren 3 und 4 sind Hinweise auf den Ort der Riechprobe 18 vorgesehen.

## Patentansprüche

1. Behälter mit einem im Behälter (15) befindlichen Produkt , wobei die Wand (1) des Behälters (15) mindestens eine Kammer (2) begrenzt, sich in der Kammer (2) eine Substanz mit einem Duftstoff (4) befindet, der durch eine Kammerwand (8) in die Umgebung des Behälters (15) diffundiert, die Kammer (2) in die Wand (1) integriert ist, das Produkt den gleichen Duftstoff (4) aufweist wie die Substanz (3), und die Kammerwand (8) eine schnellere Permeation als die Wand (1) zuläßt, **dadurch gekennzeichnet, daß** die Kammerwand (8) ein Etikett (21) ist.

2. Behälter nach Anspruch 1, **dadurch gekennzeichnet, daß** die Kammerwand (8) aus Polypropylen (6) oder Polyethylen niederer Dichte besteht.

3. Behälter nach Anspruch 2, **dadurch gekennzeichnet, daß** die Kammerwand ein gesintertes Blättchen ist.

4. Behälter nach Anspruch 1, **dadurch gekennzeichnet, daß** die Kammer (2) als Substanz (3) und Duftstoff (4) ein Parfümöl (19) in konzentrierter Form (20) enthält.

5. Behälter nach Anspruch 1 oder Anspruch 4, **dadurch gekennzeichnet, daß** die Kammerwand (8) Teil einer Verschlußkappe (24) ist.

6. Behälter nach Anspruch 1 oder Anspruch 2, **dadurch gekennzeichnet, daß** als Material (12) für die Kammerwand (8) das gleiche Material wie für die Wand (1) vorgesehen ist, und daß die Kammerwand (8) deutlich dünner als die zum Inneren (7) des Behälters (15) hin gelegene, die Kammer (2) begrenzende Wandung (14) ist.

## Claims

1. Container having a product located within the container (15), the wall (1) of the container (15) defining at least one chamber (2), a substance with a fragrance (4) being located in the chamber (2), the fragrance being diffused through a chamber wall (8) into the environment of the container (15), the chamber (2) being integrated into the wall (1), the product having the same fragrance (4) as the substance (3) and the chamber wall (8) permitting more rapid permeation than the wall (1), **characterized in that** the chamber wall (8) is a label (21).

2. Container according to Claim 1, **characterized in that** the chamber wall (8) consists of polypropylene (6) or polyethylene of relatively low density.

3. Container according to Claim 2, **characterized in that** the chamber wall is a small sintered sheet.

4. Container according to Claim 1, **characterized in that** the chamber (2) contains, as the substance (3) and fragrance (4), a perfume oil (19) in concentrated form (20).

5. Container according to Claim 1 or Claim 4, **characterized in that** the chamber wall (8) is part of a closure cap (24).

6. Container according to Claim 1 or Claim 2, **characterized in that**, as the material (12) for the chamber wall (8), the same material is provided as for the wall (1), and **in that** the chamber wall (8) is significantly thinner than the wall portion (14) defining the chamber (2) and facing towards the interior (7) of the container (15).

## Revendications

1. Récipient avec un produit contenu dans le récipient (15), la paroi (1) du récipient (15) délimitant au moins une chambre (2), une substance contenant un parfum (4) qui diffuse dans l'environnement du récipient (15) à travers une paroi (8) de la chambre étant située dans la chambre (2), la chambre (2) étant intégrée dans la paroi (1), le produit contenant le même parfum (4) que la substance (3) et la paroi (8) de la chambre permettant une perméation plus rapide que la paroi (1), **caractérisé en ce que** la paroi (8) de la chambre est une étiquette (21).

2. Récipient selon la revendication 1, **caractérisé en ce que** la paroi (8) de la chambre est réalisée en polypropylène (6) ou en polyéthylène à basse densité.

3. Récipient selon la revendication 2, **caractérisé en ce que** la paroi de la chambre est un feuillet fritté.

4. Récipient selon la revendication 1, **caractérisé en ce que** la chambre (2) contient comme substance (3) et comme parfum (4) une huile de parfum (19) sous forme concentrée (20).

5. Récipient selon la revendication 1 ou la revendication 4, **caractérisé en ce que** la paroi (8) de la chambre fait partie d'un capuchon de fermeture (24).

6. Récipient selon la revendication 1 ou la revendication 2, **caractérisé en ce qu'**en tant que matériau (12) pour la paroi (8) de la chambre est prévu le même matériau que pour la paroi (1) et **en ce que** la paroi (8) de la chambre est nettement plus mince que la paroi (14) située du côté intérieur (7) du récipient (15) et délimitant la chambre (2).
